# EUROPEAN PATENT APPLICATION

(11) **EP 2 511 366 A1**
(43) Date of publication of application: **17.10.2012**
(21) Application number: 10836038.9
(22) Date of filing: 09.12.2010
(51) Int. Cl.: C12N 5/071, A61K 35/12, A61K 35/34, A61L 27/00, A61P 9/00, C12N 5/077

(54) **AGENT FOR PROMOTING DIFFERENTIATION OF PLURIPOTENT STEM CELLS INTO CARDIAC MUSCLE CELLS WHICH INCLUDES NITROVIN**

(30) Priority: 09.12.2009 JP 2009279512
(71) Applicant: Kyoto University, Sakyo-ku Kyoto-shi Kyoto 606-8501 (JP)
(72) Inventor: NAKATSUJI, Norio, Kyoto-shi Kyoto 606-8501 (JP); UESUGI, Motonari, Kyoto-shi Kyoto 606-8501 (JP); MINAMI, Itsunari, Kyoto-shi Kyoto 606-0805 (JP); OTSUJI, Tomomi, Kyoto-shi Kyoto 606-0805 (JP)
(74) Representative: von Kreisler Selting Werner
(86) International application number: PCT/JP2010/072148
(87) International publication number: WO 2011/071118

(57) **Abstract**

The present invention provides a composition for promoting differentiation of pluripotent stem cells into cardiac muscle cells which comprises nitrovin, a method for inducing differentiation of pluripotent stem cells into cardiac muscle cells by using nitrovin and a method for preparing cardiac muscle cells by using nitrovin.

## Description

### [Technical Field]

The present invention relates to a composition for promoting differentiation of pluripotent stem cells into cardiac muscle cells, a method for inducing differentiation of pluripotent stem cells into cardiac muscle cells, and a method for preparing cardiac muscle cells from pluripotent stem cells.

### [Background Art]

A technology to induce differentiation of pluripotent stem cells into desired cells is essential for realization of regenerative medicine. In addition, cells differentiated from pluripotent stem cells are also expected to be valuable for *in vitro* drug screening study and evaluation of drug safety. In particular, it is highly desirable to develop a method to induce differentiation of pluripotent stem cells into cardiac muscle cells for regenerative medicine and drug evaluation for heart diseases because heart diseases are the second cause of death in Japan now. There are various drugs which induce severe side effects, including cardiac arrest and arrhythmia, leading to increasingly-demand to provide homogenous cardiac muscle cells which are available for cardiotoxicity study.

So far, it has been reported that cardiac muscle cell differentiation of human ES cells is induced by co-culturing human ES cells and mouse feeder cells, END2 cells (Non-patent literature 1). However, differentiation efficiency of this method is not satisfactory and it is difficult to obtain pure human cardiac muscle cells since the resulting human cardiac muscle cells are often contaminated with mouse END2 cells. It is also reported that cardiac muscle cell differentiation is induced by preparing embryoid from ES cells and adding several cytokines (fibroblast growth factor (bFGF), bone morphogenetic protein 4 (BMP4), vascular endothelial cell growth factor (VEGF), Dickkopf-1 (DKK1), Activin A) to the embryoid (Non-patent literatures 2 and 3). This method, however, requirs huge amount of cytokines and cost too much, while its differentiation efficiency is not enough.

### [Prior art references]

### [Non-patent literature]

Non-patent literature 1: Mummery, C., et al., Differentiation of human embryonic stem cells to cardiomyocytes: role of coculture with visceral endoderm-like cells. Circulation. 107(21), 2733-40 (2003).
Non-patent literature 2: Yang, L., et al., Human cardiovascular progenitor cells develop from a KDR+ embryonic-stem-cell-derived population. Nature. 453(7194), 524-8 (2008).
Non-patent literature 3: Leschik, J., et al., Cardiac commitment of primate embryonic stem cells. Nat Protoc. 3(9), 1381-7 (2008).
Those references are herein incorporated by reference.

### [Summary of Invention]

### [Problem to be Solved by the Invention]

An object of the present invention is to provide a method for preparing homogeneous cardiac muscle cells with high efficiency at low cost.

### [Means of Solving the Problem]

The present inventors examined 9,600 library compounds for the activity of promoting differentiation of monkey ES cells into cardiac muscle cells. As a results, nitrovin, which is a low molecular compound known as an antibiotic and growth promoting agent for domestic animals, was found to promote cardiac muscle cell differentiation highly efficiently compared to conventional promoters of cardiac muscle cell differentiation. Then, the present invention has been accomplished.

The present invention provides a composition for promoting differentiation of pluripotent stem cells into cardiac muscle cells which comprises nitrovin.

Further, the present invention provides a method for inducing differentiation of pluripotent stem cells into cardiac muscle cells which comprises culturing pluripotent stem cells in a medium containing nitrovin.

Further, the present invention provides a method for preparing cardiac muscle cells from pluripotent stem cells which comprises culturing pluripotent stem cells in a medium containing nitrovin.

### [Effect of the invention]

According to the present invention, it has become possible to induce differentiation of pluripotent stem cells into cardiac muscle cells without using feeder cells and then obtain pure cardiac muscle cells. In addition, according to the present invention, it has become possible to induce cardiac muscle cell differentiation with high efficiency at low cost to prepare cardiac muscle cells. The present invention is particularly useful for evaluation of QT prolongation which is critical for evaluation of drug safety in a high-throughput format, large-scale production of homogenous and mature human cardiac muscle cells for use in drug evaluation for heart diseases, and production of cardiac muscle cells for transplant to treat heart diseases.

### [Brief Description of Drawings]

[Fig. 1] Screening strategy of agents which promote cardiac muscle cell differentiation.
[Fig. 2] Screening system and detection of nitrovin.
[Fig. 3-1] GFP expression after administration of nitrovin (final concentration: 1 µM, 5 µM, 20 µM) in monkey ES cells.
[Fig. 3-2] Increase of GFP fluorescence intensity and number of beating colonies of cardiac muscle cells induced by nitrovin in monkey ES cells. The horizontal axis represents concentration of nitrovin. In the left graph, the vertical axis represents GFP fluorescence intensity after administration of nitrovin which is expressed as a percentage compared to control (dimethyl sulfoxide, DMSO) (100%). In the right graph, the vertical axis represents number of beating colonies per well.
[Fig. 4-1] GFP expression after administration of nitrovin (final concentration: 5 µM) in human ES cells.
[Fig. 4-2] Increase of GFP fluorescence intensity induced by nitrovin (final concentration: 5 µM) in human ES cells. The vertical axis represents GFP fluorescence intensity after administration of nitrovin which is expressed as a percentage compared to control (DMSO) (100%).
[Fig. 5-1] GFP expression in monkey ES cells after administration of nitrovin (final concentration: 5 µM), cytokines (bFGF, BMP4, VEGF, DKK1, and Activin A) (final concentration: 5 ng/ml, 10 ng/ml, 10 ng/ml, 150 ng/ml, and 3 ng/ml, respectively), or a combination of nitorovin (final concentration: 5 µM) and cytokines as described above.
[Fig. 5-2] Increase of GFP fluorescence intensity and number of beating colonies of cardiac muscle cells induced by nitrovin in monkey ES cells. In the left graph, the vertical axis represents GFP fluorescence intensity after administration of nitrovin and/or cytokines which is expressed as a percentage compared to control (DMSO) (100%). In the right graph, the vertical axis represents number of beating colonies per well.
[Fig. 5-3] Increase of GFP and Troponin T expression induced by nitrovin in monkey ES cells. The vertical axis represents expression level after administration of nitrovin and/or cytokines which is expressed as a percentage compared to control (DMSO) (100%).

### [Description of Embodiments]

The present invention provides a composition for promoting differentiation of pluripotent stem cells into cardiac muscle cells which comprises nitrovin. Nitrovin is a low-molecular compound having the following formula (C₁₄H₁₂N₆O₆): 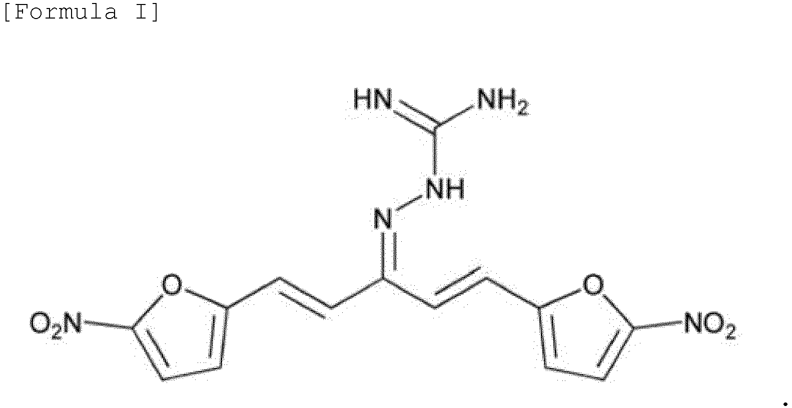 Nitovin is a known compound and may be prepared by a conventional organic synthesis method in the art. Also, nitovin may be obtained from suppliers such as KANTO CHEMICAL CO, INC (Tokyo, Japan).

The term "pluripotent stem cells" herein used refers to cells having an ability to differentiate any type of cells constituting an adult body (pluripotency) and an ability to maintain the pluripotency during cell division (self-renewal capacity). The "pluripotent stem cells" includes embryonic stem cells (ES cells), embryonic germ cells (EG cells), and induced pluripotent stem cell (iPS cells). The "pluripotent stem cells" may be cells of any species, preferably mammalian cells, and more preferably rodent or primate cells. The present invention is suitable for monkey or human pluripotent stem cells.

ES cells are pluripotent stem cells derived from early embryo and may be established from inner cell mass of a blastocyst or post-implantation epiblast in early embryo. Examples of ES cells include those described in the following references: human (Thomson J. A. et al., Science 282: 1145-1147 (1998); primates such as rhesus macaque and marmoset (Thomson J. A. et al., Proc. Natl. Acad. Sci. USA 92: 7844-7848 (1995); Thomson J. A. et al., Biol. Reprod. 55: 254-259 (1996)); rabbit (JP-A-2000-508919); hamster (Doetshman T. et al., Dev. Biol. 127: 224-227 (1988)), hog(Evans M. J. et al., Theriogenology 33: 125128 (1990); Piedrahita J.A. et al., Theriogenology 34: 879-891 (1990); Notarianni E. et al., J. Reprod. Fert. 40: 51-56 (1990); Talbot N. C. et al., Cell. Dev. Biol. 29A: 546-554 (1993)), sheep (Notarianni E. et al., J. Reprod. Fert. Suppl. 43: 255-260 (1991)), cow (Evans M. J. et al., Theriogenology 33: 125-128 (1990); Saito S. et al., Roux. Arch. Dev. Biol. 201: 134-141 (1992)), and mink (Sukoyan M. A. et al., Mol. Reorod. Dev. 33: 418-431 (1993)) (those references are herein incorporated by reference.).

EG cells are pluripotent stem cells derived from primordial germ cells. Examples of EG cells include human EG cells (Shamblott, et al., Proc. Natl. Acad. Sci USA 92: 7844-7848 (1995)).

The term "iPS cells" herein used refers to pluripotent stem cells induced from cells other than pluripotent stem cells such as somatic cells and tissue stem cells. Methods for preparing iPS cells are described in the following references, for example: WO2007/069666, WO2009/006930, WO2009/006997, WO2009/007852, WO2008/118820, Cell Stem Cell 3(5): 568-574 (2008), Cell Stem Cell 4(5): 381-384 (2009), Nature 454: 646-650 (2008), Cell 136(3) :411-419 (2009), Nature Biotechnology 26: 1269-1275 (2008), Cell Stem Cell 3: 475-479 (2008), Nature Cell Biology 11: 197-203 (2009), and Cell 133(2): 250-264 (2008)) (those references are herein incorporated by reference.). The "iPS cells" herein used should not be limited to those described in the above references. The "iPS cells" includes cells prepared by any method as long as the cells have been artificially induced from cells other than pluripotent stem cells.
When iPS cells are prepared from somatic cells, the iPS cells have the same genetic information as the somatic cells. Thus, cardiac muscle cells induced from iPS cells derived from a patient using the composition of the present invention are useful for not only regenerative medicine but also personalized evaluation of drug efficacy or side effect. In addition, cardiac muscle cells induced from iPS cells derived from a heart disease patient are expected to have genetic information representing the heart disease, and thus useful for drug screening for heart diseases caused by genetic abnormality.

The composition of the present invention may be added to a differentiation medium for cardiac muscle cell differentiation of pluripotent stem cells at a final concentration of nitrovin of 1-5 µM. The differentiation medium may be any conventional medium used for cardiac muscle cell differentiation of pluripotent stem cells and the composition of the differentiation medium is not specifically limited. Examples of the differentiation medium include the IMDM-based differentiation medium used in the examples of the present specification and DMDM-based differentiation medium consisting of 200 ml DMEM/F12 medium (Sigma), 50 ml bovine fetal serum (GIBCO), 2.5 ml MEM non-essential amino acid solution (Sigma), 2.5 ml penicillin-streptomycin (GIBCO), 2.5 ml 200 mM L-glutamine, and 2 µl 2-mercaptoethanol, and StemPro-34SFM (GIBCO) comprising BMP4(10 ng/ml). It is not necessary to use feeder cells such as END2 cells when the composition of the present invention is used. The composition of the present invention may be added to the differentiation medium at an appropriate time depending on the type of pluripotent stem cells and composition of the differentiation medium to be used. When monkey or human ES cells are cultured in the IMDM-based differentiation medium used in the examples of the present specification, the composition of the present invention may be added in the differentiation medium during day 6-14 of culture. The composition of the present invention may be used in combination with other agent(s) which promote cardiac muscle cell differentiation such as cytokines including bFGF, BMP4, VEGF, DKK1, and Activin A. The amount of the other agent (s) to be added and administration schedule of the agent(s) may be determined as appropriate.

The method for inducing cardiac muscle cell differentiation and the method for preparing cardiac muscle cells of the present invention are characterized in that pluripotent stem cells are cultured in a medium containing nitrovin. In an embodiment, the method of the present invention comprises the step of culturing pluripotent stem cells in a differentiation medium for cardiac muscle cell differentiation, adding nitrovin to the differentiation medium such that the final concentration of nitrovin is 1-5 µM during day 6-14 of culture, and confirming differentiation of the pluripotent stem cells into cardiac muscle cells at day 18 of culture. Schedule of administration of nitrovin and confirmation of cardiac muscle cell differentiation may be determined as appropriate depending on the cells to be used.
In the method of the present invention, nitrovin may be used in combination with other agent(s) which promote cardiac muscle cell differentiation such as cytokines including bFGF, BMP4, VEGF, DKK1, and Activin A. The amount of the other agent (s) to be added and administration schedule of the agent(s) may be determined as appropriate.

Culture condition of the cells may be any conventional condition for culturing animal cells such as, but not limited to, at 37°C, 100% humidity, and 5% CO₂.

Differentiation into cardiac muscle cells may be detected from the number of beating colonies of cardiac muscle cells or expression level of a maker of cardiac muscle cell differentiation such as α-MHC gene.

The cardiac muscle cells prepared by the method of the present invention may be used for evaluation of drug safety *in vitro* or as cardiac muscle cells as transplant to treat heart diseases.

### Example 1

### Screening of agents which promote cardiac muscle cell differentiation of monkey ES cells

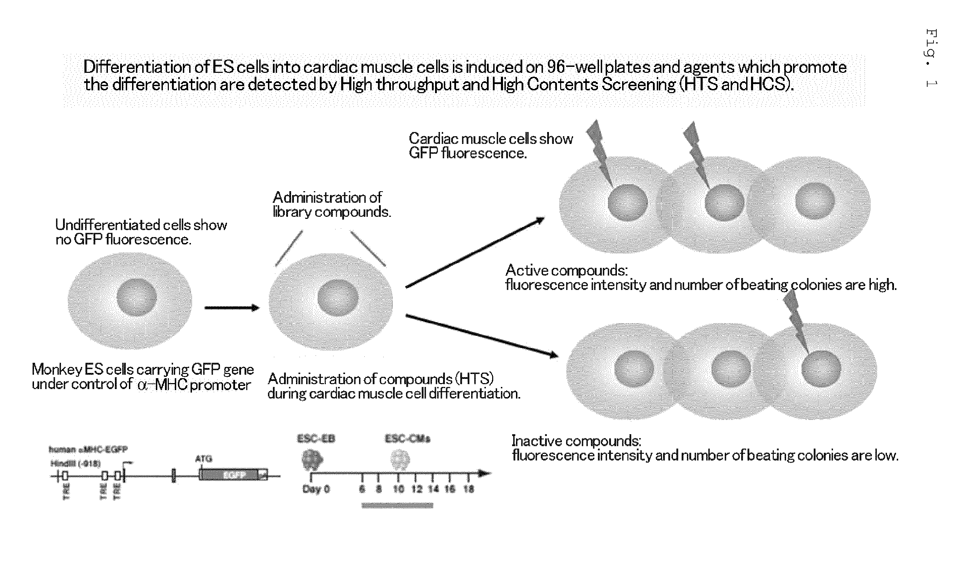

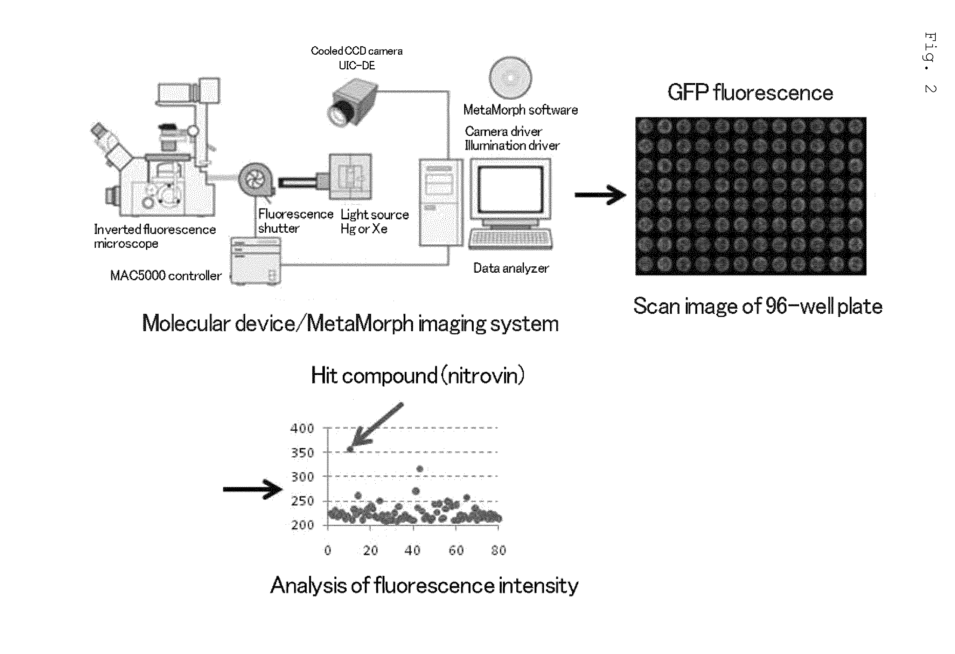

As described in Fig. 1, screening of agents which promote cardiac muscle cell differentiation of monkey ES cells was performed. A vector expressing green fluorescent protein (GFP) under control of a promoter of α-MHC gene, a marker of cardiac muscle cell differentiation, was introduced into monkey ES cell line (CMK 6.4 cynomolgus monkey ES cells) and the cells were seeded on 96-well culture plates (Greiner/655090 : 96 well FIA black plate) in 5.0 x 10³ cells/well, and cultured for 14 days in a differentiation medium for cardiac muscle cell differentiation (200 ml IMDM (Sigma 13390) containing 50 ml bovine fetal serum (GIBCO 10099-141), 2.5 ml MEM non-essential amino acid solution (Sigma M7145), 2.5 ml penicillin-streptomycin (GIBCO 15140), 2.5 ml 200 mM L-glutamine, 2 µl 2-mercaptoethanol (Sigma M7522), 255 µl 5N NaOH 255 µl). During day 6-14 of culture, 9,600 library compounds were added into separate wells (about 1-5 µM compound/well). Then, at day 14 of culture, GFP expression was determined by using HCS (high contents screening) system (Molecular device/MetaMorph imaging system). As a result, GFP fluorescence intensity was high in the wells added with nitrovin (Fig. 2).

### Example 2

### Effect of nitrovin for promoting cardiac muscle cell differentiation of monkey ES cells

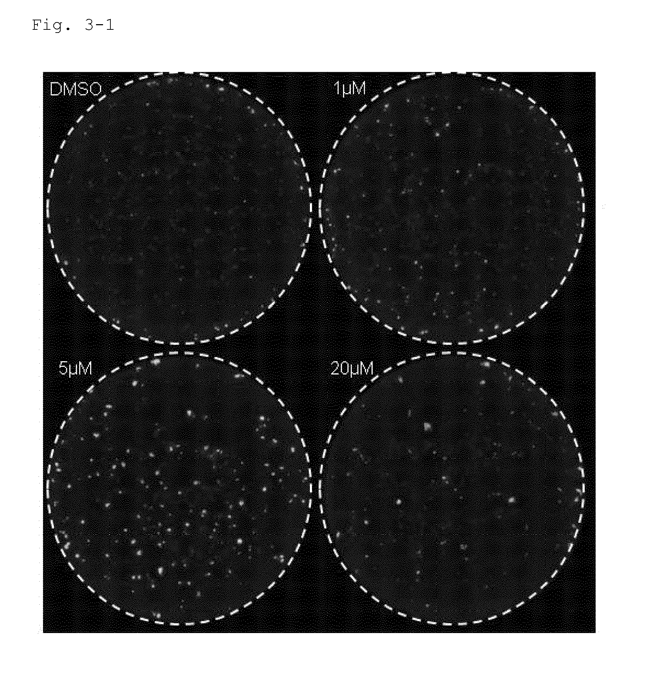

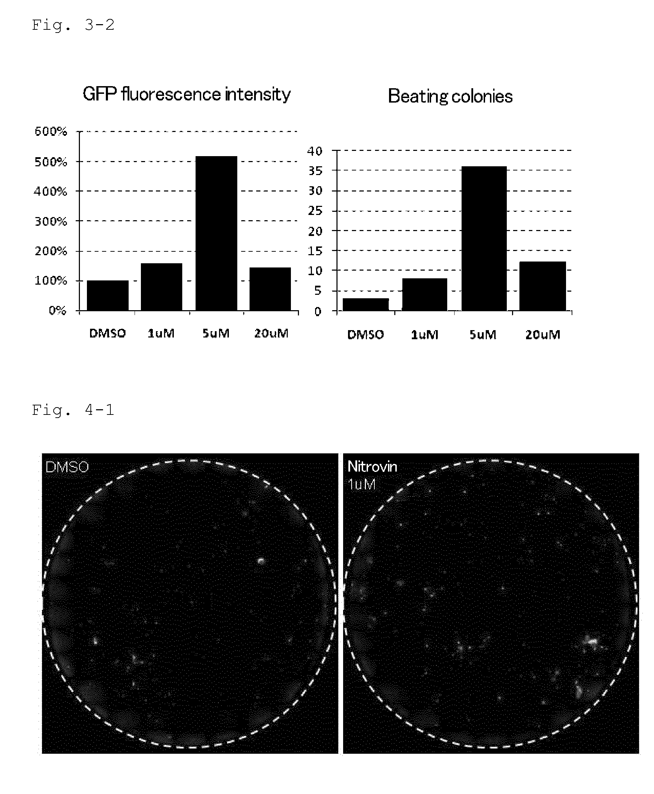

As described in Example 1, a vector expressing green fluorescent protein (GFP) was introduced into monkey ES cell line (CMK 6.4 cynomolgus monkey ES cells). The monkey ES cells were seeded and cultured on 6-well culture plates (Asahi Glass/5816-006: Ezview culture plate) in 4.0 x 10⁵ cells/well. The culture medium was the same as described in Example 1. At day 6 of culture, nitrovin was added at a final concentration of 1 µM, 5 µM, or 20 µM. At day 18 of culture, fluorescence intensity of GFP and number of beating colonies of cardiac muscle cells were determined. When nitrovin was added, the fluorescence intensity and number of beating colonies increased up to about 5 times and about 10 times higher, respectively (Fig 3-1 and 3-2). The final concentration of 5 µM was most effective.

### Example 3

### Effect of nitrovin for promoting cardiac muscle cell differentiation of human ES cells

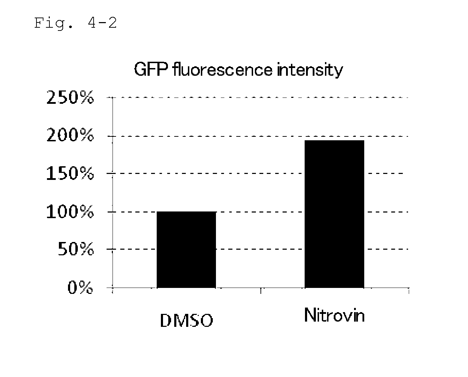

As described in Example 1, a vector expressing green fluorescent protein (GFP) was introduced into human ES cell line (Kh-1). The human ES cells were seeded and cultured on 6-well culture plates (Asahi Glass/5816-006: Ezview culture plate) in 1.2 x 10⁶ cells/well. The culture medium was the same as described in Example 1. At day 6 of culture, nitrovin was added at a final concentration of 1 µM, 5 µM, or 20 µM. At day 18 of culture, fluorescence intensity of GFP was determined. As a result, increase of GFP fluorescence intensity induced by nitrovin was also observed in human ES cells (Fig 4-1 and 4-2).

### Example 4

### Comparison of nitrovin and cytokines

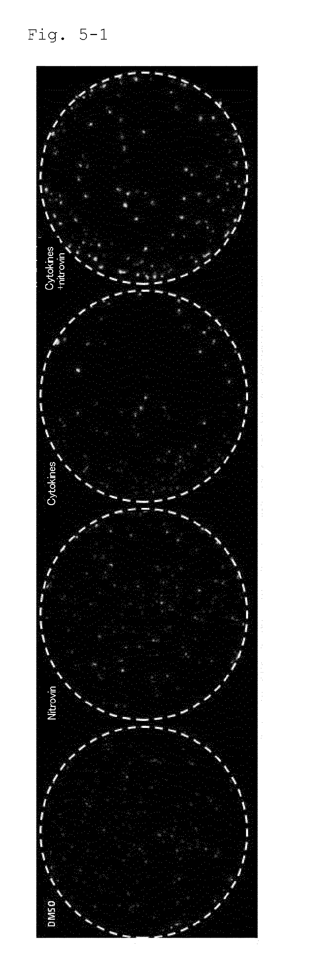

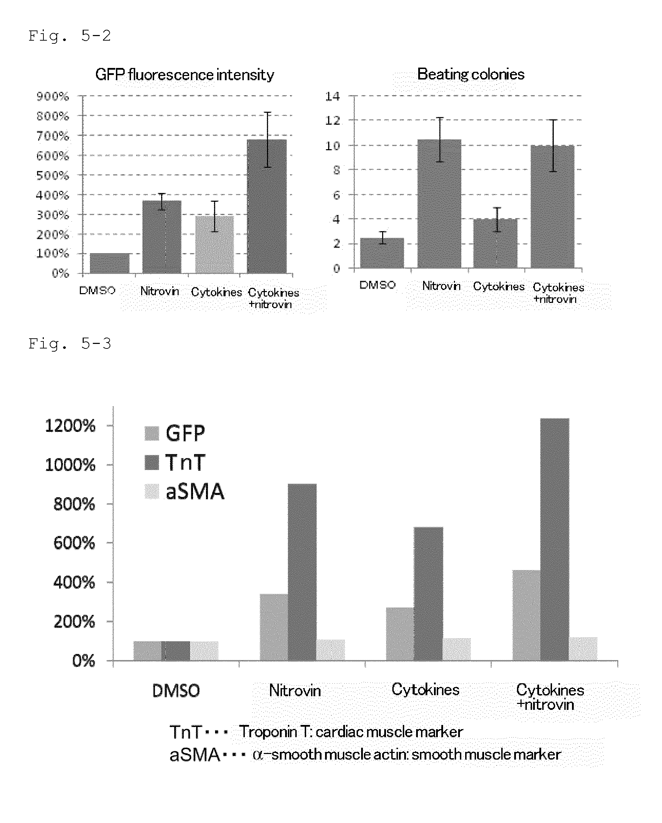

Nitrovin and a mixture of cytokines (bFGF, BMP4, VEGF, DKK1, and Activin A) known as a promoter of cardiac muscle cell differentiation were compared as to increase of GFP fluorescence intensity, number of beating colonies of cardiac muscle cells, and expression of Troponin T, a marker of cardiac muscle cell differentiation.
As described in Example 1, a vector expressing green fluorescent protein (GFP) was introduced into monkey ES cell line (CMK 6.4 cynomolgus monkey ES cells). The culture medium was the same as described in Example 1. The monkey ES cells were seeded on 6-well culture plates and nitrovin (final concentration: 5 µM) or a mixture of cytokines (bFGF, BMP4, VEGF, DKK1, and Activin A) (final concentration: 5 ng/ml, 10 ng/ml, 10 ng/ml, 150 ng/ml, and 3 ng/ml, respectively) was added during day 6-14 of culture (nitrovin) or day 1-14 of culture (cytokines). At day 18 of culture, GFP fluorescence intensity, number of beating colonies of cardiac muscle cells, and expression level of Troponin T were determined. Compared to the mixture of cytokines, nitrovin was highly effective on any of GFP fluorescence intensity, number of beating colonies, and expression level of Troponin T (Fig. 5-1 to 5-3). In addition, combination of nitrovin and cytokines showed a synergistic effect on the increase of GFP fluorescence intensity and number of beating colonies (Fig. 5-1 and 5-3).

## Claims

1. A composition for promoting differentiation of pluripotent stem cells into cardiac muscle cells which comprises nitrovin.

2. The composition according to Claim 1, wherein the pluripotent stem cells are mammalian cells.

3. The composition according to Claim 2, wherein the pluripotent stem cells are primate cells.

4. The composition according to Claim 3, wherein the pluripotent stem cells are human cells.

5. A method for inducing differentiation of pluripotent stem cells into cardiac muscle cells which comprises culturing pluripotent stem cells in a medium containing nitrovin.

6. A method for preparing cardiac muscle cells from pluripotent stem cells which comprises culturing pluripotent stem cells in a medium containing nitrovin.

7. Cardiac muscle cells prepared by the method of Claim 6.
